# FASCICULE DE BREVET EUROPEEN

(11) **EP 1 232 743 B1**
(45) Date de publication et mention de la délivrance du brevet: **26.04.2006**
(21) Numéro de dépôt: 02290337.1
(22) Date de dépôt: 12.02.2002
(51) Int. Cl.: A61K 8/19, A61K 8/44, A61K 8/34, A61K 8/37

(54) **Composition cosmétique et/ou dermatologique procurant un effet de refraîchissement**
Erfrischende kosmetische und/oder dermatologische Zusammensetzung
Refreshing cosmetic and/or dermatological composition

(30) Priorité: 14.02.2001 FR 0102007
(43) Date de publication de la demande: 21.08.2002
(73) Titulaire: Laboratoire Nuxe, 75010 Paris (FR)
(72) Inventeur: Leclere, Jacques, 45500 Saint-Gondon (FR)
(74) Mandataire: L'Helgoualch, Jean

(56) Documents cités:
- EP-A- 0 303 792
- EP-A- 0 419 148
- WO-A-99/00109
- FR-A- 2 771 632
- FR-A- 2 779 059
- DATABASE WPI Section Ch, Week 199011 Derwent Publications Ltd., London, GB; Class B05, AN 1990-082444 XP002182170 & SU 1 486 168 A (BIOKHIMREAKTIV COMB), 15 juin 1989 (1989-06-15)
- PATENT ABSTRACTS OF JAPAN vol. 010, no. 120 (C-343), 6 mai 1986 (1986-05-06) & JP 60 244335 A (TOUYOU BIYUUTEI KK), 4 décembre 1985 (1985-12-04)
- PATENT ABSTRACTS OF JAPAN vol. 005, no. 094 (C-059), 19 juin 1981 (1981-06-19) & JP 56 039007 A (HOOU KK), 14 avril 1981 (1981-04-14)
- DATABASE WPI Section Ch, Week 198419 Derwent Publications Ltd., London, GB; Class A96, AN 1984-119008 XP002182172 & SU 1 033 138 A (NIKOLAEV ALYE PARUSA), 7 août 1983 (1983-08-07)

## Description

La présente invention concerne l'utilisation cosmétique en tant qu'agent rafraîchissant de l'aspartate de magnésium pour la fabrication d'une composition cosmétique et l'utilisation de l'aspartate de magnésium pour la fabrication d'une composition dermatologique procurant un effet rafraîchissant.

Dans la préparation de compositions cosmétiques, et tout particulièrement dans les compositions pour apaiser et/ou protéger des peaux sensibles, pour hydrater des peaux sèches et/ou ralentir le vieillissement de la peau, on connaît l'utilisation d'aspartate de magnésium et d'aspartate de potassium. Ce type de préparation est en particulier décrit dans le document FR-A-2.771.632. Le brevet SU-A-1486168 décrit une composition cosmétique contenant un mélange d'asparaginate de potassium, d'asparaginate de magnésium, d'acides aminés et d'hydrolysats de protéines ayant pour but de diminuer les effets secondaires des acides aminés. L'utilisation d'aspartate de potassium et de magnésium pour améliorer la stabilité d'une composition cosmétique à base d'huile d'olive est décrite dans le brevet SU-A-1033138.

Notamment on connaît de l'enseignement du document FR-A-2.779.059, une composition cosmétique pour lutter contre les effets du vieillissement cutané mettant en oeuvre un sel de métal divalent, par exemple l'aspartate de magnésium. Dans ce cas, l'aspartate de magnésium est utilisé comme agent favorisant l'adhésion des kératinocytes de la couche basale épidermique à la jonction dermo-épidermique. Une telle composition renfermant cet agent d'adhésion est présentée comme permettant de diminuer la profondeur des rides, de ralentir leur apparition, et/ou de restaurer la tonicité et l'élasticité de la peau.

L'aspartate de magnésium est également connu dans les compositions cosmétiques pour avoir un effet défatiguant et déstressant des couches supérieures de l'épiderme.

Dans l'état de la technique, on connaît également des compositions cosmétiques qui donnent une sensation de froid lorsqu'elles sont appliquées sur la peau. Ces compositions peuvent comporter par exemple du menthol ou des produits dérivés du menthol, ou bien encore des produits à structure à base de menthol tel que le menthoxypropanediol. En général, une telle sensation de froid est recherchée pour les compositions prévues pour tonifier les tissus de la peau. Cette sensation de froid peut être également utile dans les compositions dermatologiques apaisantes, notamment pour des applications topiques ou externes à titre d'après-soleil. Cette sensation de froid est recherchée pour apporter un certain confort à l'utilisateur.

Cependant, les compositions cosmétiques et dermatologiques comportant du menthol ou un de ses dérivés, créent localement des sensations d'inconfort, et d'irritation au niveau des zones sur lesquelles la composition a été appliquée. En effet, le menthol a un effet de froid très puissant au niveau cutané. De plus, le menthol présente également d'autres inconvénients.

Par exemple, on observe fréquemment des vasoconstrictions locales et superficielles de l'épiderme, suivies généralement de vasodilatations pour rétablir une température normale de la peau. Ce type de réaction génère un effet hyperhémiant inconfortable. Les vasodilatations créent des rougeurs cutanées au niveau où la composition a été appliquée. Dans le cas où la composition cosmétique est un lait démaquillant, l'apparition de ces rougeurs notamment sur le pourtour des yeux présente un caractère rédhibitoire.

Il apparaît que les compositions apportant un effet de froid posent un problème dans la mesure où parmi les sensations détectées par les utilisateurs, certaines peuvent être source d'inconfort. L'intérêt de l'invention, est qu'elle propose une composition cosmétique et/ou dermatologique apportant un effet de fraîcheur. L'effet de fraîcheur se distingue significativement de l'effet de froid. Il est moins brutal, plus superficiel, et, de plus, ne génère pas d'irritation, ni de vasodilatation ou de vasoconstriction au niveau de la peau sur laquelle la composition présentant cet effet de fraîcheur est appliquée.

A cet effet, la demanderesse a découvert que la présence d'aspartate de magnésium dans les compositions cosmétiques et/ou dermatologiques procure cet effet de fraîcheur en évitant les inconvénients du menthol.

L'invention a pour objet l'utilisation de l'aspartate de magnésium pour la fabrication d'une composition dermatologique procurant un effet rafraîchissant et l'utilisation cosmétique en tant qu'agent rafraîchissant de l'aspartate de magnésium pour la fabrication de composition cosmétique.

Dans un mode de réalisation préféré, la composition comprend une proportion d'aspartate de magnésium comprise entre 0,1 et 10 % en poids par rapport au poids total de la composition, et de préférence entre 0,1 et 5 % en poids de la composition, et plus préférentiellement entre 0,2 et 1,0 %.

Outre l'aspartate de magnésium, la composition contient avantageusement du gamma oryzanol. Le gamma oryzanol peut notamment être obtenu à partir d'huile de son de riz. Le gamma oryzanol, connu pour ses capacités à stimuler les phénomènes de circulation sanguine tout en gardant un effet hyperhémiant, permet de potentialiser l'effet de fraîcheur procuré par la présence d'aspartate de magnésium dans la composition. De préférence, le gamma oryzanol représente entre 0 et 5% en poids de la composition, et plus particulièrement environ 0,1%.

Suivant une caractéristique préférentielle de la présente invention, la préparation comprend un ou plusieurs alcools gras. Dans ce cas, la préparation est préférentiellement une émulsion. Cette émulsion se trouve sous forme liquide ou semi-liquide, telle que sa viscosité est relativement faible. Ainsi l'émulsion peut être facilement appliquée sur une surface cutanée de grande envergure.

L'alcool gras peut être choisi parmi les alcools gras naturels ou synthétiques à chaîne aliphatique linéaire ou ramifiée, et par exemple parmi les alcools béhénique, butylique, stéarylique, caprylique, laurylique, myristilique, cétéarylique, oléylique et cétylique. Ces alcools peuvent être utilisés isolément ou en mélange.

Selon une autre caractéristique préférentielle de l'invention, la préparation comprend une phase grasse dans une proportion comprise entre 3 et 40 % en poids, dans le cas des émulsions. Les matières constituant la phase grasse peuvent être choisies parmi les alcools gras, les triglycérides et les huiles végétales. Les huiles végétales peuvent notamment être choisies parmi l'huile de jojoba, l'huile de maïs, l'huile vaseline, l'huile de coco hydrogénée, l'huile de carthame, l'huile de Coprah, hydrogénée ou non, l'huile de noisettes, et l'huile de bourrache. Les huiles peuvent encore être choisies parmi les huiles de silicone telles que la diméthicone, la cyclométhicone, et la cyclométhicone/diméthicone copolyol. Outre des huiles végétales, la composition peut comporter des esters, des hydrocarbures, du squalane, des silicones, des cires, ou encore de la lécithine.

Les alcools gras, qui sont inclus dans les matières grasses, représentent généralement entre 0,5 et 8 % en poids par rapport au poids total de l'émulsion.

Il peut aussi être avantageux d'incorporer dans la composition un actif complémentaire destiné à améliorer le comportement de la peau, tel que le tocophérol. Le tocophérol, ou un dérivé tel que l'acétate de tocophérol, est tout particulièrement utile en raison de sa capacité à piéger les radicaux libres et à empêcher l'oxydation des corps gras de l'émulsion.

La composition peut en outre contenir divers adjuvants et excipients couramment utilisés dans les techniques des compositions cosmétiques et dermatologiques. Elle peut par exemple contenir des conservateurs, des épaississants, des gélifiants hydrophiles ou lipophiles, des agents émulsionnants, des agents viscosants, des antioxydants, des agents hydratants, des tensioactifs, des parfums, et divers additifs destinés à améliorer les propriétés physiques de la composition.

Les gélifiants ou épaississants peuvent par exemple être choisis parmi les copolymères d'acrylate et d'acide acrylique, les dérivés de cellulose comme des mucilages, notamment un mucilage d'acacia angustifolia, ou des gommes naturelles.

L'agent hydratant ou humectant peut être choisi parmi un polyol, le sorbitol, le maltitol, le pentaérythritol, la glycérine, le glycol, le phénoxyéthanol, les polyacrylates et polyméthacrylates de glycéryle, le glycérol ou des dérivés du glycérol. D'une manière générale, tout agent hydratant convenant aux compositions cosmétiques ou dermatologiques peut être utilisé dans la présente invention. L'agent humectant est avantageusement introduit dans la phase aqueuse lors de la préparation de l'émulsion. La teneur en agent humectant est généralement comprise entre 0,1 et 5 % en poids par rapport au poids total de l'émulsion, mais elle peut représenter jusqu'à 20 %, et même 30 % en poids par rapport au poids total de l'émulsion, notamment lorsque les agents humectants sont choisis parmi des polyols ou des glycols, par exemple le butylène glycol, ou la glycérine.

Les conservateurs usuels de la technique des compositions cosmétiques ou dermatologiques peuvent être utilisés dans l'invention, et par exemple des alcools tels que l'alcool éthylique, l'alcool benzylique et l'isopropanol, ou la benzophénone, le phénoxyéthanol, l'acide benzoïque et des parahydroxybenzoates de méthyle et/ou de propyle, isolément ou en combinaison.

Des agents de protection contre les rayons ultraviolets peuvent également être utilisés, en particulier quand la composition contient des dérivés vinyliques et acryliques sensibles à la lumière. Ces agents de protection peuvent par exemple être des filtres solaires tels que la benzophénone ou un dérivé de benzophénone, ou un cinnamate et plus particulièrement le méthoxycinnamate d'octyle (Parsol®).

Des agents de coloration peuvent également être incorporés. Par exemple, on peut prévoir d'ajouter des nacres ou du titane pour obtenir un effet nacré spécifique.

Les compositions peuvent se présenter sous la forme de crèmes, d'émulsions huile-dans-eau (H/E) ou d'émulsions eau-dans-huile (E/H), de crèmes, de laits, de gels. Ces diverses formes galéniques sont préparées suivant les techniques usuelles. Ces compositions peuvent être présentées sous forme de produits de soins ou de maquillage, et par exemple de gel hydratant, ou d'émulsion démaquillante.

Les caractéristiques et avantages de la présente invention apparaîtront plus en détail dans la description qui suit, relative à des formes préférentielles de réalisation. Dans ces exemples, les parties et pourcentages sont exprimés en poids, sauf indication contraire.

### Exemple 1

On prépare un gel pour le visage en opérant comme indiqué ci dessous, en utilisant les techniques usuelles de l'industrie des produits cosmétiques et dermatologiques. La composition pondérale est la suivante.

| Phase A: | |
|---|---|
| Eau déminéralisée | 48,00 g |
| Benzophénone 4® | 0,10 g |
| Polymère sous forme d'émulsion: | |
| Acrylates/ Palmeth 25 - acrylate copolymère (solution à 30%) | 2,00 g |

| Phase B: | |
|---|---|
| Diméthicone copolyol | 8,00 g |
| Cyclométhicone | 5,00 g |
| Polymère de diméthicone | 3,00 g |
| PEG 1500 | 3,00 g |
| Glycereth 7 | |
| (glycérine à 7 mol d'oxyde d'éthylène) | 5,00 g |
| Phase C: | |
| Pastilles d'hydroxyde de potassium pur | 0,16 g |
| Eau déminéralisée | 6,00 g |
| Phase D: | |
| Nacres de Mica + | 4,40 g |
| Titane - méthylméthacrylate réticulé | 3,00 g |
| Polyméthylsilsesquioxane | 3,00 g |
| Nylon 12 | 5,00 g |
| Phase E: | |
| Mucilage d'Acacia Angustifolia | 3,00 g |
| Phase F: | |
| Extrait de THEA Chinensis | 0,50 g |
| Phase G: | |
| Aspartate de magnésium | 0,25 g |
| Phase H: | |
| Alcool | 2,00 g |
| Phénoxyéthanol | 0,40 g |
| Parahydroxybenzoate de méthyle | 0,30 g |
| Parfum | 0,50 g |

Les produits de la phase A sont mélangés à température ambiante. Après homogénéisation de la phase B, celle-ci est incorporée dans la phase A. De même, la phase C est homogénéisée avant son incorporation dans le mélange précédent. La phase C permet de neutraliser les composants de la phase A.

Chacune des phases D, E, F, G et H est homogénéisée, si nécessaire, avant leur incorporation successive à température ambiante dans le mélange principal. La phase F comprend généralement un antiradicalaire, présentant par exemple des fonctions polyphénoliques.

La composition obtenue avec l'exemple 1 sert préférentiellement à obtenir un gel hydratant.

### Exemple 2

On prépare dans cet exemple une émulsion ayant la composition indiquée ci-après.

| Phase A: | |
|---|---|
| Alcool gras | 6,50 g |
| Huiles végétales | 7,50 g |
| Perhydrosqualène | 3,40 g |
| Gamma oryzanol | 0,10 g |
| Tocophérol | 1,20 g |
| Phase B: | |
| Butylène glycol-1,3 | 3,00 g |
| Phénoxyéthanol | 0,40 g |
| Mélange de parahydroxybenzoates | 0,30 g |
| EDTA sodique | 0,10 g |
| Eau QSP 100 | |
| Phase C: | |
| Acide citrique (solution à 10%) | 0,10 g |
| Phase D: | |
| Aspartate de magnésium | 0,50 g |
| Eau déminéralisée | 2,00 g |
| Phase E: | |
| Extrait de Persea Gratissima | 4,00 g |
| Phase F: | |
| Parfum | 0,15 g |

Les composants de la phase A sont chauffés au bain-marie à 75°C. La phase B est chauffée à 77°C jusqu'à homogénéisation, puis est introduite dans la phase A. De l'eau est rajouter dans le mélange de la phase A et de la phase B pour atteindre une masse de 100 grammes du mélange total. Ensuite, on laisse refroidir ce mélange, et on ajoute la phase C lorsque le mélange précédent est redescendu à la température de 40°C. Les composants des phases D, E, F sont incorporés au mélange précédent, lorsque celui-ci atteint la température de 35°C. La phase E contient du Persea Gratissima qui permet d'améliorer les fonctions cutanées.

On laisse ensuite refroidir à température ambiante. Cette émulsion peut être utilisée comme lait démaquillant.

## Revendications

1. Utilisation cosmétique en tant qu'agent rafraîchissant de l'aspartate de magnésium pour la fabrication d'une composition cosmétique.

2. Utilisation de l'aspartate de magnésium pour la fabrication d'une composition dermatologique procurant un effet rafraîchissant.

3. Utilisation selon la revendication 1 ou 2,
**caractérisée en ce que** la composition comprend du gamma oryzanol.

4. Utilisation selon l'une quelconque des revendications précédentes, **caractérisée en ce que** la composition comprend un ou plusieurs alcools gras.

5. Utilisation selon la revendication 4, **caractérisée en ce que** les alcools gras de la composition sont choisis parmi les alcools gras naturels ou synthétiques à chaîne aliphatique linéaire ou ramifiée, et par exemple parmi les alcools béhénique, butylique, stéarylique, caprylique, laurylique, myristilique, cétéarylique, oléylique et cétylique.

6. Utilisation selon l'une quelconque des revendications 1 à 5, **caractérisée en ce que** l'aspartate de magnésium représente entre 0,1 et 10 % en poids de la composition.

7. Utilisation selon la revendication 6, **caractérisée en ce que** l'aspartate de magnésium représente entre 0,1 et 5 % en poids de la composition, préférentiellement entre 0,2 % et 1,0 %.

8. Utilisation selon la revendication 3, **caractérisée en ce que** le gamma oryzanol représente entre 0 et 5% en poids de la composition.

9. Utilisation selon la revendication 8, **caractérisée en ce que** le gamma oryzanol représente 0,1% en poids de la composition.

10. Utilisation selon l'une quelconque des revendications 1 à 9, **caractérisée en ce que** la composition comprend du tocophérol ou des dérivés du tocophérol.

## Patentansprüche

1. Kosmetische Verwendung von Magnesiumaspartat als Erfrischungsmittel zur Herstellung einer kosmetischen Zusammensetzung.

2. Verwendung von Magnesiumaspartat zur Herstellung einer dermatologischen Zusammensetzung, die eine erfrischende Wirkung ausübt.

3. Verwendung nach Anspruch 1 oder 2, **dadurch gekennzeichnet, dass** die Zusammensetzung γ-Oryzanol umfasst.

4. Verwendung nach einem der vorangehenden Ansprüche, **dadurch gekennzeichnet, dass** die Zusammensetzung einen oder mehrere Fettalkohole umfasst.

5. Verwendung nach Anspruch 4, **dadurch gekennzeichnet, dass** die Fettalkohole der Zusammensetzung aus natürlichen oder synthetischen Fettalkoholen mit unverzweigter oder verzweigter aliphatischer Kette, beispielsweise aus Behen-, Butyl-, Stearyl-, Capryl-, Lauryl-, Myristyl-, Cetearyl-, Oleyl- und Cetylalkohol, ausgewählt sind.

6. Verwendung nach einem der Ansprüche 1 bis 5, **dadurch gekennzeichnet, dass** das Magnesiumaspartat zwischen 0,1 und 10 Gew.-% der Zusammensetzung ausmacht.

7. Verwendung nach Anspruch 6, **dadurch gekennzeichnet, dass** das Magnesiumaspartat zwischen 0,1 und 5 Gew.-% der Zusammensetzung, vorzugsweise zwischen 0,2 % und 1,0 %, ausmacht.

8. Verwendung nach Anspruch 3, **dadurch gekennzeichnet, dass** das γ-Oryzanol zwischen 0 und 5 Gew.-% der Zusammensetzung ausmacht.

9. Verwendung nach Anspruch 8, **dadurch gekennzeichnet, dass** das γ-Oryzanol 0,1 Gew.-% der Zusammensetzung ausmacht.

10. Verwendung nach einem der Ansprüche 1 bis 9, **dadurch gekennzeichnet, dass** die Zusammensetzung Tocopherol oder Derivate von Tocopherol umfasst.

## Claims

1. Cosmetic use of magnesium aspartate as refreshing agent for the manufacture of a cosmetic composition.

2. Use of magnesium aspartate for the manufacture of a dermatological composition providing a refreshing effect.

3. Use according to claim 1 or 2, **characterized in that** the composition comprises gamma oryzanol.

4. Use according to any of the preceding claims, **characterised in that** the composition comprises one or several fatty alcohols.

5. Use according to claim 4, **characterized in that** the fatty alcohols of the composition are selected from the natural or synthetic fatty alcohols with straight or branched aliphatic chain, and for example from behenic, butylic, stearylic, caprylic, laurylique, myristylic, cetearylic, oleylic and cetylic alcohols.

6. Use according to any of claims 1 to 5, **characterized in that** the magnesium aspartate represents between 0.1 and 10% by weight of the composition.

7. Use according to claim 6, **characterized in that** the magnesium aspartate represents between 0.1 and 5% by weight of the composition, preferably between 0.2 and 1%.

8. Use according to claim 3, **characterized in that** the gamma oryzanol represents between 0 and 5% by weight of the composition.

9. Use according to claim 8, **characterized in that** the gamma oryzanol represents 0.1% by weight of the composition.

10. Use according to any of claims 1 to 9, **characterized in that** the composition comprises tocopherol or tocopherol derivatives.
